(19) Europäisches Patentamt
European Patent Office
Office européen des brevets

(11) Veröffentlichungsnummer: **0 227 938 B1**

(12) # EUROPÄISCHE PATENTSCHRIFT

(45) Veröffentlichungstag der Patentschrift: **15.04.92**

(51) Int. Cl.[5]: **C12P 21/02**, C12N 15/62, C12N 1/20

(21) Anmeldenummer: **86116140.4**

(22) Anmeldetag: **21.11.86**

Ein Antrag gemäss Regel 88 EPÜ auf Berichtung der Figuren 1 und 2 liegt vor. Uber diesen Antrag wird im Laufe des Verfahrens von der Prüfungsabteilung eine Entscheidung getroffen werden.

(54) **Eukaryotische Fusionsproteine, ihre Herstellung und Verwendung sowie Mittel zur Durchführung des Verfahrens.**

(30) Priorität: **27.11.85 DE 3541856**

(43) Veröffentlichungstag der Anmeldung:
**08.07.87 Patentblatt 87/28**

(45) Bekanntmachung des Hinweises auf die Patenterteilung:
**15.04.92 Patentblatt 92/16**

(84) Benannte Vertragsstaaten:
**AT BE CH DE ES FR GB GR IT LI LU NL SE**

(56) Entgegenhaltungen:
**EP-A- 0 155 655**
**EP-A- 0 158 198**
**EP-A- 0 158 564**
**EP-A- 0 171 024**
**EP-A- 0 211 299**

(73) Patentinhaber: **HOECHST AKTIENGESELLSCHAFT**
**Postfach 80 03 20**
**W-6230 Frankfurt am Main 80(DE)**

(72) Erfinder: **Habermann, Paul, Dr.**
**Heimchenweg 80**
**W-6230 Frankfurt am Main 80(DE)**
Erfinder: **Wengenmayer, Friedrich, Dr.**
**Am Seyenbach 38**
**W-6238 Hofheim am Taunus(DE)**

**EP 0 227 938 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung bezieht sich auf einen "offenen Leseraster" aus einer DNA, die für Interleukin-2 codiert, und die Verwendung dieser DNA als Expressionshilfe zur Expression von Peptiden bzw. Proteinen.

Bei der gentechnischen Herstellung eukaryotischer Proteine wird in Bakterien häufig nur eine geringe Ausbeute erhalten, insbesondere bei kleinen Proteinen mit einem Molgewicht bis zu etwa 15 000 Dalton, deren Strukturen Disulfidbrücken enthalten. Man nimmt an, daß die gebildeten Proteine durch wirtseigene Proteasen rasch abgebaut werden. Man konstruiert deshalb zweckmäßig Genstrukturen, die für Fusionsproteine codieren, wobei der unerwünschte Anteil des Fusionsproteins ein wirtseigenes Protein ist, das nach der Isolation der Primärproduktes nach an sich bekannten Methoden abgespalten wird.

Es wurde nun überraschenderweise gefunden, daß ein N-terminaler Anteil von Interleukin-2, der im wesentlichen den ersten 100 Aminosäuren entspricht, besonders gut zur Herstellung von Fusionsproteinen geeignet ist. Man erhält also als Primärprodukt ein Fusionsprotein, das völlig oder zum ganz überwiegenden Teil aus eukaryotischen Proteinsequenzen besteht. Überraschenderweise wird dieses Protein offenbar in dem betreffenden Wirtsorganismus nicht als Fremdprotein erkannt und nicht sofort wieder abgebaut. Ein weiterer Vorteil ist, daß die erfindungsgemäßen Fusionsproteine schwer löslich bis unlöslich sind und sich somit einfach, zweckmäßig durch Zentrifugation, von den löslichen Proteinen abtrennen lassen.

Da es erfindungsgemäß hinsichtlich der Funktion als "Ballast-Anteil" des Fusionsproteins nicht darauf ankommt, daß der Interleukin-2-Anteil ein biologisch aktives Molekül darstellt, kommt es insofern auch nicht auf die exakte Struktur des Interleukin-2-Anteils an. Es genügt hierfür, daß im wesentlichen die ersten 100 N-terminalen Aminosäuren vorliegen. Es ist also beispielsweise möglich, am N-Terminus Variationen vorzunehmen, die eine Spaltung des Fusionsproteins erlauben, falls das erwünschte Protein N-terminal dazu angeordnet ist. Umgekehrt kann man C-terminal Variationen vornehmen, um die Abspaltung des gewünschten Proteins zu ermöglichen oder zu erleichtern, falls dieses im Fusionsprotein - wie üblich - C-terminal gebunden ist.

Die für Human-Interleukin-2, im folgenden "IL-2", codierende natürliche DNA-Sequenz ist aus der europäischen Patentanmeldung mit der Veröffentlichungsnummer EP-A1-0 091 539 bekannt. Die dort aufgeführte Literatur bezieht sich auch auf Mäuse- und Ratten-IL-2. Diese Säuger-DNA kann zur Synthese der erfindungsgemäßen Proteine herangezogen werden. Zweckmäßiger geht man jedoch von einer synthetischen DNA aus, besonders vorteilhaft von der DNA für Human-IL-2, die in der (nicht vorveröffentlichten) deutschen Offenlegungsschrift 34 19 995 (entsprechend der unter der Nummer 0 163 249 veröffentlichten europäischen Patentanmeldung) vorgeschlagen wurde. Diese synthetische DNA-Sequenz ist im Anhang wiedergegeben (DNA-Sequenz I). Diese synthetische DNA hat nicht nur den Vorzug, daß sie in der Codon-Wahl auf die Gegebenheiten des am häufigsten verwendeten Wirts, E. coli, abgestimmt ist, sondern sie enthält auch eine Reihe von Schnittstellen für Restriktionsendonucleasen, von denen erfindungsgemäß Gebrauch gemacht werden kann. In der folgenden Tabelle 1 ist eine Auswahl der geeigneten Schnittstellen am Anfang bzw. in der Region des 100. Tripletts wiedergegeben. Hierdurch ist jedoch nicht ausgeschlossen, daß in dem dazwischenliegenden Bereich Variationen in der DNA vorgenommen werden, wobei von den in der vorstehend genannten Patentanmeldung aufgeführten weiteren Schnittstellen Gebrauch gemacht werden kann.

Tabelle 1

| Restriktionsenzym | Erkennungssequenz 5' 3' | Position des ersten Nucleotids der Erkennungssequenz (codierender Strang) |
|---|---|---|
| Aha II, Ban I, Hae II, Nar I, | GGCGCC | 8 |
| Ban II, Sac I, Sst I | GAGCTC | 291 |
| Hha I | GCGC | 9 |
| Hinf I | GACTC | 35 |
| Pvu I | CGATCG | 346 |
| Taq I | TCGA | 387 |

Wird von den Nucleasen Ban II, Sac I oder Sst I Gebrauch gemacht, so erhält man eine IL-2-Teilsequenz, die für etwa 95 Aminosäuren codiert. Diese Länge ist im allgemeinen ausreichend, um ein unlösliches Fusionsprotein zu erhalten. Wenn die Schwerlöslichkeit, beispielsweise bei einem gewünschten hydrophilen eukaryotischen Protein, noch nicht ausreicht, man aber - um so wenig "Ballast" wie möglich zu produzieren - nicht von den näher am C-Terminus liegenden Schnittstellen Gebrauch machen will, so kann man durch entsprechende Adapter bzw. Linker die DNA-Sequenz am N-und/oder C-terminalen Ende verlängern und so den "Ballast"-Anteil "maßschneidern". Man kann natürlich auch die DNA-Sequenz - mehr oder weniger - bis zum Ende nutzen und so - gegebenenfalls modifiziertes - biologisch aktives IL-2 als "Nebenprodukt" erzeugen bzw. ein bifunktionelles Protein erzeugen, das IL-2 Wirkung zusätzlich zur Wirkung des codierten Proteins zeigt.

Die Erfindung betrifft somit Fusionsproteine der allgemeinen Formel

$$\text{Met} - X - Y - Z \quad (\text{Ia}) \qquad \text{oder} \qquad \text{Met} - Z - Y - X \quad (\text{Ib})$$

in der X im wesentlichen die Aminosäurefolge der etwa 100 ersten Aminosäuren von vorzugsweise menschlichem IL-2 bedeutet, Y eine direkte Bindung bedeutet, falls die zum gewünschten Protein benachbarte Aminosäure oder Aminosäurenfolge eine Abspaltung des gewünschten Proteins ermöglicht, oder andernfalls ein Brückenglied aus einer oder mehreren genetisch codierbaren Aminosäuren, das die Abspaltung ermöglicht, und Z eine Sequenz aus genetisch codierbaren Aminosäuren ist, die für das gewünschte Protein codiert.

Wie sich aus den Formeln Ia und Ib ergibt - und wie es auch schon vorstehend erwähnt wurde - ist es möglich, das gewünschte Protein vor oder nach dem IL-2-Anteil zur Expression zu bringen. Zur Vereinfachung wird im folgenden im wesentlichen die erste Möglichkeit erläutert, die der herkömmlichen Methode zur Herstellung von Fusionsproteinen entspricht. Wenn also im folgenden diese "klassische" Variante beschrieben wird, soll die andere Alternative hierdurch nicht ausgeschlossen werden.

Die Spaltung des Fusionsproteins kann in an sich bekannter Weise chemisch oder enzymatisch erfolgen. Die Wahl der geeigneten Methode richtet sich vor allem nach der Aminosäuresequenz des gewünschten Proteins. Wenn dieses beispielsweise kein Methionin enthält, kann Y Met bedeuten, worauf eine chemische Spaltung mit Chlor- oder Bromcyan erfolgt. Steht im Bindeglied Y am Carboxyterminus Cystein oder steht Y für Cys, so kann eine enzymatische Cysteinspezifische Spaltung oder eine chemische Spaltung, beispielsweise nach spezifischer S-Cyanylierung, folgen. Steht im Brückenglied Y am Carboxyterminus Tryptophan oder Y für Trp, so kann eine chemische Spaltung mit N-Bromsuccinimid erfolgen.

Proteine, die in ihrer Aminosäuresequenz nicht
Asp - Pro
enthalten und hinreichend säurestabil sein, können in an sich bekannter Weise proteolytisch gespalten werden. Hierdurch erhält man Proteine, die N-terminal Prolin bzw. C-terminal Asparaginsäure enthalten. Auf diese Weise können also auch modifizierte Proteine synthetisiert werden.

Die Asp-Pro-Bindung kann noch säurelabiler gestaltet werden, wenn dieses Brückenglied $(Asp)_n$-Pro bzw. Glu-$(Asp)_n$-Pro ist, wobei n 1 bis 3 bedeutet.

Beispiele für enzymatische Spaltungen sind ebenfalls bekannt, wobei auch modifizierte Enzyme mit verbesserter Spezifität eingesetzt werden können (vgl. C.S. Craik et al., Science 228 (1985) 291-297). Ist das gewünschte eukaryotische Peptid Proinsulin, so wählt man zweckmäßig als Sequenz Y eine Peptidsequenz, bei der eine durch Trypsin abspaltbare Aminosäure (Arg, Lys) an die N-terminale Aminosäure (Phe) des Proinsulins gebunden ist, beispielsweise Ala-Ser-Met-Thr-Arg, da dann die Arginin-spezifische Spaltung mit der Protease Trypsin erfolgen kann.

Enthält das gewünschte Protein nicht die Aminosäurefolge
Ile-Glu-Gly-Arg,
so kann das Fusionsprotein mit Faktor Xa gespalten werden (europäische Patentanmeldungen mit den Veröffentlichungsnummern 0 025 190 und 0 161 973).

Das Fusionsprotein wird durch Expression in einem geeigneten Expressionssystem in an sich bekannter Weise gewonnen. Hierfür eignen sich alle bekannten Wirts-Vektor-Systeme, also beispielsweise Säugerzellen und Mikroorganismen, beispielsweise Hefen und vorzugsweise Bakterien, insbesondere E. coli.

Die DNA-Sequenz, die für das gewünschte Protein codiert, wird in bekannter Weise in einen Vektor eingebaut, der in dem gewählten Expressionssystem eine gute Expression gewährleistet.

In bakteriellen Wirten wählt man zweckmäßig den Promotor und Operator aus der Gruppe lac, tac, trp, $P_L$ oder $P_R$ des Phagen $\lambda$, hsp, omp oder einen synthetischen Promotor, wie sie beispielsweise in der deutschen Offenlegungsschrift 34 30 683 (Europäische Patentanmeldung mit der Veröffentlichungsnummer 0 173 149) vorgeschlagen sind. Vorteilhaft ist die tac Promotor-Operator-Sequenz, die inzwischen handelsüblich ist (z.B. Expressionsvektor pKK223-3, Pharmacia, "Molecular Biologicals, Chemicals and Equipment for Molecular Biology", 1984, S. 63).

Bei der Expression des erfindungsgemäßen Fusionsproteins kann es sich als zweckmäßig erweisen, einzelne Tripletts der ersten Aminosäuren nach dem ATG-Start-Codon zu verändern, um eine eventuelle Basenpaarung auf der Ebene der mRNA zu verhindern. Solche Veränderungen, ebenso wie Veränderungen, Deletionen oder Additionen einzelner Aminosäuren im IL-2-Proteinanteil, sind dem Fachmann geläufig und ebenfalls Gegenstand der Erfindung.

In den folgenden Beispielen und in den Figuren wird die Erfindung näher erläutert. Hierbei beziehen sich

Figur 1 und deren Fortsetzung, Figur 1a, auf die Synthese. des Plasmids pK360, das für ein Fusionsprotein codiert, welches die Hirudinsequenz aufweist;

Figur 2 und ihre Fortsetzung, Figur 2a, betreffen die Synthese des Plasmids pK410, welches ebenfalls für ein Fusionsprotein mit der Aminosäuresequenz des Hirudin codiert,

Figur 3 und ihre Fortsetzungen, Figuren 3a bis 3c, auf die Konstruktion der Plasmide pPH15, 16, 20 und 30, die für Fusionsproteine codieren, die die Aminosäuresequenz von Affen-Proinsulin enthalten,

Figur 4 auf die Synthese des Plasmids pPH100, welches für ein Fusionsprotein mit der Aminosäuresequenz des Hirudin codiert,

Figur 5 und ihre Fortsetzung, Figur 5a, auf die Konstruktion des Plasmids pK370, welches für ein Fusionsprotein mit der Aminosäuresequenz des Hirudin codiert sowie

Figur 6 und ihre Fortsetzung, Figur 6a, auf die Synthese des Plasmids pKH101, das für ein Fusionsprotein mit der Aminosäurefolge von Affenproinsulin codiert.

Die Figuren sind i.a. nicht maßstabgerecht gezeichnet, vor allem bei der Wiedergabe der Polylinker wurde der Maßstab "gedehnt".

## Beispiel 1

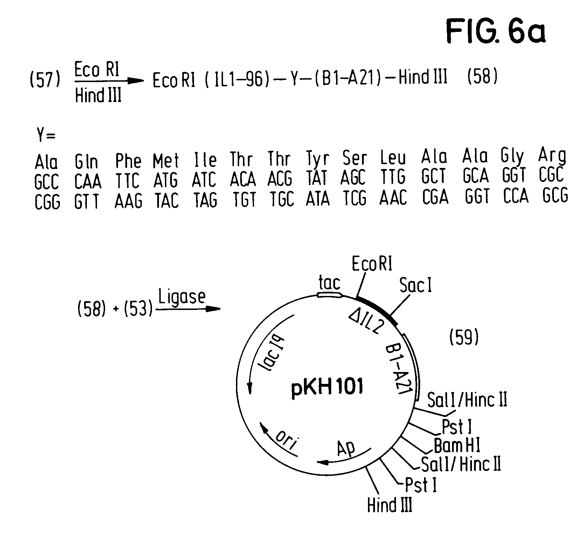

Durch Insertion des lac-Repressors (P.J. Farabaugh, Nature 274 (1978) 765-769) in das Plasmid pKK 177-3 (Amann et al., Gene 25 (1983) 167) erhält man das Plasmid pJF118 (1) (Fig. 1; vgl. deutsche Patentanmeldung P 35 26 995.2, Beispiel 6, Fig. 6). Dieses wird an der singulären Restriktionsstelle für Ava I geöffnet und in an sich bekannter Weise durch Exonuclease-Behandlung um etwa 1000 bp verkleinert.

Nach Ligierung wird das Plasmid pEW 1000 (2), (Figur 1) erhalten, in dem das lac-Repressorgen vollständig erhalten ist, das aber auf Grund der Verkleinerung in deutlich höherer Kopienzahl als das Ausgangsplasmid vorliegt.

Anstelle des Plasmids pKK177-3 kann man auch von dem vorstehend erwähnten handelsüblichen Plasmid pKK223-3 ausgehen, den lac-Repressor einbauen und das erhaltene Produkt analog verkürzen.

Das Plasmid pEW 1000 (2) wird mit den Restriktionsenzymen EcoR I und Sal I geöffnet (3).

Das für Hirudin codierende Plasmid (4), hergestellt gemäß deutscher Offenlegungsschrift 34 29 430 (europäische Patentanmeldung mit der Veröffentlichungsnummer 0 171 024), Beispiel 4 (Figur 3), wird mit den Restriktionsenzymen Acc I und Sal I geöffnet und das kleine Fragment (5), das zum größten Teil die Hirudin-Sequenz enthält, isoliert.

Das Plasmid p159/6 (6), hergestellt gemäß deutscher Offenlegungsschrift 34 19 995 (europäische Patentanmeldung mit der Veröffentlichungsnummer 0 163 249), Beispiel 4 (Figur 5), wird mit den Restriktionsenzymen Eco RI und Pvu I geöffnet und das kleine Fragment (7) isoliert, das den größten Teil der IL-2-Sequenz enthält. Diese Teilsequenz und im folgenden auch andere verkürzte IL-2 Sequenzen sind in den Figuren mit "ΔIL2" bezeichnet.

Anschließend werden die Sequenzen (3), (5), (7) sowie die synthetische DNA-Sequenz (8; Figur 1a) mit T4-Ligase behandelt. Man erhält das Plasmid pK360 (9).

Kompetente E. coli-Zellen werden mit dem Ligationsprodukt transformiert und auf NA-Platten, die 25 µg/ml Ampicillin enhalten, ausplattiert. Die Plasmid-DNA der Klone wird mittels Restriktions- und Sequenzanalyse charakterisiert.

Eine Übernachtkultur aus E. coli-Zellen, die das Plasmid (9) enthalten, wird mit LB-Medium (J. H. Miller, Experiments in Molecular Genetics, Cold Spring Harbor Laboratory, 1972), das 50 µg/ml Ampicillin enthält, im Verhältnis von etwa 1:100 verdünnt und das Wachstum über OD-Messung verfolgt. Bei OD = 0,5 wird die Schüttelkultur auf 1 mM Isopropyl-β-galactopyranosid (IPTG) eingestellt und die Bakterien nach 150 bis 180 Minuten abzentrifugiert. Die Bakterien werden 5 Minuten in einer Puffermischung (7M Harnstoff, 0,1% SDS, 0,1 M Natriumphosphat, pH 7,0) gekocht und Proben auf eine SDS-Gelelektrophoreseplatte aufgetragen. Nach Elektrophorese wird aus Bakterien, die das Plasmid (9) enthalten, eine Proteinbande erhalten, die der Größe des erwarteten Fusionsproteins entspricht. Nach Aufschluß der Bakterien (French Press; (R)Dyno-Mühle) und Zentrifugation befindet sich das Fusionsprotein im Niederschlag, so daß mit dem Überstand bereits erhebliche Mengen der übrigen Proteine abgetrennt werden können. Nach Isolierung des Fusionsproteins wird durch Bromcyan-Spaltung das erwartete Hirudin-Peptid freigesetzt. Dieses wird nach Isolierung durch Protein-Sequenzanalyse charakterisiert.

Die angegebenen Induktionsbedingungen gelten für Schüttelkulturen; bei größeren Fermentationen sind entsprechend veränderte OD-Werte und gegebenenfalls leicht variierte IPTG-Konzentrationen zweckmäßig.

## Beispiel 2

Das Plasmid (4) (Figur 1) wird mit Acc I geöffnet und die überstehenden Enden mit Klenow-Polymerase aufgefüllt. Anschließend wird mit Sac I geschnitten und das Fragment (10) isoliert, das den größten Teil der Hirudin-Sequenz enthält.

Der handelsübliche Vektor pUC 13 wird mit den Restriktionsenzymen Sac I und Sma I geöffnet und das große Fragment (11) isoliert.

Mittels T4-Ligase werden nun die Fragmente (10) und (11) zum Plasmid pK 400 (12) (Fig. 2) ligiert. Das Plasmid (12) ist in der Figur 2 zweimal dargestellt, wobei in der unteren Darstellung die Aminosäuresequenz des so erhältlichen Hirudin-Derivats hervorgehoben wird.

Das Plasmid (4) (Figur 1) wird mit den Restriktionsenzymen Kpn I und Sal I geöffnet und das kleine Fragment (13) isoliert, das die Hirudin-Teilsequenz enthält.

Das Plasmid (12) wird mit den Restriktionsenzymen Hinc II und Kpn I umgesetzt und das kleine Fragment (14) isoliert, das die Hirudin-Teilsequenz enthält.

Das Plasmid (9) (Figur 1a) wird mit EcoR I partiell gespalten, die freien Enden mit Klenow-Polymerase in einer fill-in-Reaktion aufgefüllt und mit Sal I geschnitten. Man erhält das Derivat des Plasmids pK360 (15).

Durch Ligieren der Fragmente (3), (13), (14) und (15) erhält man das Plasmid pK410 (16), das in der Figur 2a zweifach dargestellt ist, wobei die untere Wiedergabe die Aminosäurefolge des Fusionsproteins und damit des nach Säurespaltung erhaltenen Hirudin-Derivats erkennen läßt.

Nach Expression und Aufarbeitung gemäß Beispiel 1 erhält man ein neues Hirudin-Derivat, das in den Positionen 1 und 2 die Aminosäuren Prolin und Histidin aufweist. Dieses Hirudin-Derivat zeigt die gleiche Aktivität wie das Naturprodukt gemäß deutscher Offenlegungsschrift 34 29 430, das in diesen Positionen die Aminosäuren Threonin und Tyrosin aufweist, ist jedoch stabiler gegen den Angriff von Aminopeptidasen, woraus sich Vorteile bei der in-vivo-Anwendung ergeben können.

Beispiel 3

Der handelsübliche Vektor pBR 322 wird mit Bam H I geöffnet, wobei man das linearisierte Plasmid (17) erhält. Die freien Enden werden partiell unter Einsatz von dATP, dGTP und dTTP aufgefüllt und das überstehende Nucleotid G mit S1-Nuclease abgebaut, wobei das pBR 322-Derivat (18) erhalten wird.

Das Hae III-Fragment (19) aus Affen-Proinsulin (Wetekam et al., Gene 19 (1982) 181) wird mit dem modifizierten Plasmid (18) ligiert, wobei das Plasmid pPH 1 (20) erhalten wird. Da die Insulin-Teilsequenz in das Tetracyclin-Gen eingesetzt wurde, sind die Klone, die dieses Plasmid enthalten, nicht gegen Tetracyclin resistent und können so identifiziert werden.

Das Plasmid (20) wird mit Bam HI und Dde I geöffnet und das kleine Fragment (21) isoliert.

Zusätzlich wird aus der Affen-Proinsulinsequenz die Dde I-Pvu II-Teilsequenz (22) isoliert.

Der Vektor pBR 322 wird mit Bam HI und Pvu II geöffnet und das linearisierte Plasmid (23) isoliert.

Durch Ligierung der Insulin-Teilsequenzen (21) und (22) mit dem geöffneten Plasmid (23) erhält man das Plasmid pPH5 (24). Dieses wird mit Bam HI und Pvu II geöffnet und das kleine Fragment (25) isoliert.

Zur Ergänzung der Insulinstruktur wird die DNA-Sequenz (26) synthetisiert.

Der handelsübliche Vektor pUC 8 wird mit den Enzymen Bam HI und Sal I geöffnet und das Restplasmid (27) isoliert. Dieses wird mit den DNA-Sequenzen (25) und (26) zum Plasmid pPH 15 (28) ligiert. Dieses wird mit Sal I geöffnet und die überstehenden Enden aufgefüllt. Aus dem resultierenden Plasmidderivat (29) wird mit Bam HI die DNA-Sequenz (30) abgespalten.

Der handelsübliche Vektor pUC 9 wird mit den Enzymen Bam HI und Sma I geöffnet und das große Fragment (31) isoliert. Dieses wird mit der DNA-Sequenz (30) ligiert, wobei das Plasmid pPH16 (32) erhalten wird.

Das Plasmid (32) wird mit Sal I geöffnet und das linearisierte Plasmid (33) partiell mit dCTP, dGTP und dTTP aufgefüllt und das verbleibende Nucleotid T mit S1-Nuclease abgespalten. Das so erhaltene Plasmidderivat (34) wird mit Bam HI behandelt und aus dem Produkt (35) mit S1-Nuclease der überstehende Einzelstrang entfernt, wobei das Plasmidderivat (36) erhalten wird.

Die stumpfen Enden des Plasmidderivats (35) werden zum Plasmid pPH 20 (37) cyclisiert.

Kompetente E. coli Hb 101-Zellen werden mit dem Ligationsgemisch transformiert und auf selektivem Medium ausplattiert. Klone, die das gewünschte Plasmid enthalten, exprimieren Proinsulin, wobei von 70 getesteten Klonen 28 radioimmunologisch nachweisbares Proinsulin enthielten. Die Plasmide werden ferner mittels DNA-Sequenzanalyse charakterisiert. Sie enthalten DNA, die vor dem Codon für die erste Aminosäure der B-Kette (Phe) für Arginin codiert.

Das Plasmid (37) wird mit Hind III gespalten, die überstehenden Enden aufgefüllt und mit Dde I nachgespalten. Das kleine Fragment (38) wird isoliert.

Das Plasmid (28) (Figur 3a) wird mit Sal I und Dde I gespalten und das kleine Fragment (39) abgetrennt.

Das Plasmid (9) (Figur 1a) wird zunächst mit Acc I gespalten, die freien Enden aufgefüllt und mit Eco RI partiell nachgespalten. Das Fragment (40), das die verkürzte IL-2-Sequenz enthält, wird isoliert.

Das linearisierte Plasmid (3) (Figur 1) und die DNA-Segmente (38), (39) und (40) werden nunmehr zu dem Plasmid pPH 30 (41) ligiert. Dieses Plasmid codiert für ein Fusionsprotein, das im Anschluß an die Aminosäuren 1 bis 114 des IL-2 die folgende Aminosäuresequenz aufweist:

Asp-Phe-Met-Ile-Thr-Thr-Tyr-Ser-Leu-Ala-Ala-Gly-Arg.

Das Arginin als letzte Aminosäure dieses Brückengliedes Y ermöglicht die Abspaltung der Insulinketten mit Trypsin.

Ausgehend vom Plasmid (9) (Figur 1a) kann man auch auf folgendem Weg zum Plasmid (41) kommen: Man öffnet (9) mit Acc I, füllt die überstehenden Enden auf, schneidet mit Sal I nach und ligiert das erhaltene Plasmidderivat (42) mit den Segmenten (3), (38) und (39).

Beispiel 4

Das Plasmid (6) (Figur 1) wird mit den Restriktionsenzymen Taq I und Eco RI geöffnet und das kleine Fragment (43) isoliert. Dieses Fragment wird mit der synthetisierten DNA-Sequenz (44) und den Segmenten (3) und (5) zum Plasmid pPH 100 (45) ligiert. Dieses Plasmid codiert für ein Fusionsprotein, bei dem auf die ersten 132 Aminosäuren des IL-2 das Brückenglied Asp-Pro und hierauf die Aminosäurefolge von Hirudin folgt. Die proteolytische Spaltung liefert somit ein modifiziertes, biologisch aktives IL-2', in dem in Position 133 an Stelle von Thr Asp enthalten ist, und ein Hirudin-Derivat, das N-terminal Pro vor der Aminosäuresequenz des Naturprodukts enthält. Auch dieses Produkt ist biologisch aktiv und im Vergleich zu dem Naturprodukt stabiler gegen den Angriff von Proteasen.

Das IL-2'-Hirudin-Fusionsprotein zeigt ebenfalls biologische Aktivität:
Im Zellproliferationstest mit einer IL-2-abhängigen Zell-linie (CTLL2) wurde biologische Aktivität gefunden.

Nach Denaturierung in 6 M Guanidiniumhydrochlorid-Lösung und anschließende Renaturierung in Pufferlösung (10 mM Tris-HCl, pH 8,5, 1 mM EDTA) wurde weiterhin hohe IL-2-Aktivität gefunden. Außerdem wurde die Gerinnungszeit von säurebehandeltem, mit Thrombin versetzem Blut nach Zugabe des Fusionsproteins verlängert.

Man erhält somit ein bifunktionelles Fusionsprotein.

Beispiel 5

Der handelsübliche Vektor pUC 12 wird mit den Restriktionsenzymen Eco RI und Sac I geöffnet. In dieses linearisierte Plasmid (46) wird eine IL-2-Teilsequenz eingesetzt, die aus dem Plasmid (6) (Figur 1) mit den Restriktionsenzymen Eco RI und Sac I herausgespalten wird. Diese Sequenz (47) umfaßt die kompletten Tripletts für die ersten 94 Aminosäuren des IL-2. Durch Ligieren von (46) und (47) erhält man das Plasmid pK 300 (48).

Das Plasmid (9) (Figur 1a) wird mit Eco RI geöffnet, die überstehenden Enden aufgefüllt und mit Hind III nachgespalten. Man isoliert das kleine Fragment (49), das im Anschluß an die für Hirudin codierende DNA-Sequenz einen Teil des Polylinkers aus pUC 12 enthält.

Das Plasmid (48) wird mit den Restriktionsenzymen Sma I und Hind III geöffnet und das große Fragment (50) isoliert. Durch Ligierung von (50) mit (49) erhält man das Plasmid pK 301 (51).

Mit dem Ligationsgemisch werden kompetente E. coli 294-Zellen transformiert. Klone, die das Plasmid (51) enthalten, werden durch Restriktionsanalyse charakterisiert. Sie enthalten DNA, die im Anschluß an die Codons für die ersten 96 Aminosäuren des IL-2 Codons für ein Brückenglied von 6 Aminosäuren und darauf folgend die Codons für Hirudin enthalten.

Das Plasmid (51) wird mit Eco RI und Hind III umgesetzt und das Fragment (52) isoliert, das die DNA-Sequenz für das genannte eukaryotische Fusionsprotein enthält.

Das Plasmid (2) (Figur 1) wird mit Eco RI und Hind III geöffnet. Das erhaltene linearisierte Plasmid (53) wird mit der DNA-Sequenz (52) ligiert, wobei das Plasmid pK 370 (54) erhalten wird.

Wird das Plasmid (54) entsprechend Beispiel 1 in E. coli zur Expression gebracht, so erhält man ein Fusionsprotein, bei dem auf die ersten 96 Aminosäuren des IL-2 das Brückenglied
Ala-Gln-Phe-Met-Ile-Thr
und im Anschluß daran die Aminosäurefolge des Hirudin folgt.

Beispiel 6

Aus dem Plasmid (41) (Beispiel 3; Figur 3c) wird mit den Restriktionsenzymen Eco RI und Hind III das DNA-Segment herausgespalten, das für Affenproinsulin codiert, und die überstehenden Enden aufgefüllt. Man erhält das DNA-Segment (55).

Das Plasmid (48) (Beispiel 5, Figur 5) wird mit Sma I geöffnet und mit Alkalischer Rinderphosphatase behandelt. Das so erhaltene linearisierte Plasmid (56) wird mit dem DNA-Segment (55) ligiert, wobei das Plasmid pK 302 (57) erhalten wird. E. coli 294-Zellen werden mit dem Ligationsgemisch transformiert, wobei Klone, die das gewünschte Plasmid enthalten, zunächst durch Restriktions- und dann durch Sequenzanalyse der Plasmid-DNA charakterisiert werden.

Aus dem Plasmid (57) wird mit Eco RI und Hind III das Segment (58) herausgespalten, das für IL-2 und Affenproinsulin codiert.

Das Plasmid (2) (Beispiel 1, Figur 1) wird ebenfalls mit Eco RI und Hind III gespalten und in das linearisierte Plasmid (3) das Segment (58) hineinligiert. Man erhält das Plasmid pKH 101 (59).

Die Expression gemäß Beispiel 1 führt zu einem Fusionsprotein, bei dem auf die ersten 96 Aminosäuren des IL-2 ein Brückenglied mit 14 Aminosäuren folgt (entsprechend Y im DNA-Segment (58)), woran sich die Aminosäurefolge des Affenproinsulins anschließt.

Anhang I: DNA-Sequenz des Interleukin-2

| | | | | | | |
|---|---|---|---|---|---|---|
| Triplett Nr. | | | | 0 | 1 | 2 |
| Aminosäure | | | | Met | Ala | Pro |
| Nucleotid Nr. | | 1 | | | 10 | |
| Cod. Strang | 5' | AA | TTC | ATG | GCG | CCG |
| nicht cod. Strang | 3' | | G | TAC | CGC | GGC |

| | | | | | | | | | |
|---|---|---|---|---|---|---|---|---|---|
| 3 | 4 | 5 | 6 | 7 | 8 | 9 | 10 | 11 | 12 |
| Thr | Ser | Ser | Ser | Thr | Lys | Lys | Thr | Gln | Leu |
| | 20 | | | | 30 | | | 40 | |
| ACC | TCT | TCT | TCT | ACC | AAA | AAG | ACT | CAA | CTG |
| TGG | AGA | AGA | AGA | TGG | TTT | TTC | TGA | GTT | GAC |

| | | | | | | | | | |
|---|---|---|---|---|---|---|---|---|---|
| 13 | 14 | 15 | 16 | 17 | 18 | 19 | 20 | 21 | 22 |
| Gln | Leu | Glu | His | Leu | Leu | Leu | Asp | Leu | Gln |
| | 50 | | | | 60 | | | 70 | |
| CAA | CTG | GAA | CAC | CTG | CTG | CTG | GAC | CTG | CAG |
| GTT | GAC | CTT | GTG | GAC | GAC | GAC | CTG | GAC | GTC |

| | | | | | | | | | |
|---|---|---|---|---|---|---|---|---|---|
| 23 | 24 | 25 | 26 | 27 | 28 | 29 | 30 | 31 | 32 |
| Met | Ile | Leu | Asn | Gly | Ile | Asn | Asn | Tyr | Lys |
| | 80 | | | | 90 | | | 100 | |
| ATG | ATC | CTG | AAC | GGT | ATC | AAC | AAC | TAC | AAA |
| TAC | TAG | GAC | TTG | CCA | TAG | TTG | TTG | ATG | TTT |

| | | | | | | | | | |
|---|---|---|---|---|---|---|---|---|---|
| 33 | 34 | 35 | 36 | 37 | 38 | 39 | 40 | 41 | 42 |
| Asn | Pro | Lys | Leu | Thr | Arg | Met | Leu | Thr | Phe |
| | 110 | | | | 120 | | | 130 | |
| AAC | CCG | AAA | CTG | ACG | CGT | ATG | CTG | ACC | TTC |
| TTG | GGC | TTT | GAC | TGC | GCA | TAC | GAC | TGG | AAG |

| | | | | | | | | | |
|---|---|---|---|---|---|---|---|---|---|
| 43 | 44 | 45 | 46 | 47 | 48 | 49 | 50 | 51 | 52 |
| Lys | Phe | Tyr | Met | Pro | Lys | Lys | Ala | Thr | Glu |
| | 140 | | | | 150 | | | 160 | |
| AAA | TTC | TAC | ATG | CCG | AAA | AAA | GCT | ACC | GAA |
| TTT | AAG | ATG | TAC | GGC | TTT | TTT | CGA | TGG | CTT |

| | | | | | | | | | |
|---|---|---|---|---|---|---|---|---|---|
| 53 | 54 | 55 | 56 | 57 | 58 | 59 | 60 | 61 | 62 |
| Leu | Lys | His | Leu | Gln | Cys | Leu | Glu | Glu | Glu |
| | 170 | | | | 180 | | | 190 | |
| CTG | AAA | CAC | CTC | CAG | TGT | CTA | GAA | GAA | GAG |
| GAC | TTT | GTG | GAG | GTC | ACA | GAT | CTT | CTT | CTC |

| | | | | | | | | | |
|---|---|---|---|---|---|---|---|---|---|
| 63 | 64 | 65 | 66 | 67 | 68 | 69 | 70 | 71 | 72 |
| Leu | Lys | Pro | Leu | Glu | Glu | Val | Leu | Asn | Leu |
| | 200 | | | | 210 | | | 220 | |
| CTG | AAA | CCG | CTG | GAG | GAA | GTT | CTG | AAC | CTG |
| GAC | TTT | GGC | GAC | CTC | CTT | CAA | GAC | TTG | GAC |

| | | | | | | | | | |
|---|---|---|---|---|---|---|---|---|---|
| 73 | 74 | 75 | 76 | 77 | 78 | 79 | 80 | 81 | 82 |
| Ala | Gln | Ser | Lys | Asn | Phe | His | Leu | Arg | Pro |
| | 230 | | | | 240 | | | 250 | |
| GCT | CAG | TCT | AAA | AAT | TTC | CAC | CTG | CGT | CCG |
| CGA | GTC | AGA | TTT | TTA | AAG | GTG | GAC | GCA | GGC |

| | | | | | | | | | |
|---|---|---|---|---|---|---|---|---|---|
| 83 | 84 | 85 | 86 | 87 | 88 | 89 | 90 | 91 | 92 |
| Arg | Asp | Leu | Ile | Ser | Asn | Ile | Asn | Val | Ile |
| | 260 | | | | 270 | | | 280 | |
| CGT | GAC | CTG | ATC | TCT | AAC | ATC | AAC | GTT | ATC |
| GCA | CTG | GAC | TAG | AGA | TTG | TAG | TTG | CAA | TAG |

| | | | | | | | | | |
|---|---|---|---|---|---|---|---|---|---|
| 93 | 94 | 95 | 96 | 97 | 98 | 99 | 100 | 101 | 102 |
| Val | Leu | Glu | Leu | Lys | Gly | Ser | Glu | Thr | Thr |
| | 290 | | | | 300 | | | 310 | |
| GTT | CTG | GAG | CTC | AAA | GGT | TCT | GAA | ACC | ACG |
| CAA | GAC | CTC | GAG | TTT | CCA | AGA | CTT | TGG | TGC |

| | | | | | | | | | |
|---|---|---|---|---|---|---|---|---|---|
| 103 | 104 | 105 | 106 | 107 | 108 | 109 | 110 | 111 | 112 |
| Phe | Met | Cys | Glu | Tyr | Ala | Asp | Glu | Thr | Ala |
| | 320 | | | | 330 | | | 340 | |
| TTC | ATG | TGC | GAA | TAC | GCG | GAC | GAA | ACT | GCG |
| AAG | TAC | ACG | CTT | ATG | CGC | CTG | CTT | TGA | CGC |
| 113 | 114 | 115 | 116 | 117 | 118 | 119 | 120 | 121 | 122 |
| Thr | Ile | Val | Glu | Phe | Leu | Asn | Arg | Trp | Ile |
| | 350 | | | | 360 | | | 370 | |
| ACG | ATC | GTT | GAA | TTT | CTG | AAC | CGT | TGG | ATC |
| TGC | TAG | CAA | CTT | AAA | GAC | TTG | GCA | ACC | TAG |
| 123 | 124 | 125 | 126 | 127 | 128 | 129 | 130 | 131 | 132 |
| Thr | Phe | Cys | Gln | Ser | Ile | Ile | Ser | Thr | Leu |
| | 380 | | | | 390 | | | 400 | |
| ACC | TTC | TGC | CAG | TCG | ATC | ATC | TCT | ACC | CTG |
| TGG | AAG | ACG | GTC | AGC | TAG | TAG | AGA | TGG | GAC |
| 133 | 134 | 135 | | | | | | | |
| Thr | | | | | | | | | |
| | 410 | | | | | | | | |
| ACC | TGA | TAG | | | 3' | | | | |
| TGG | ACT | ATC | AGC | T | 5' | | | | |

## Patentansprüche

### Patentansprüche für folgende Vertragsstaaten : BE, CH, DE, FR, GB, GR, IT, LI, LU, NL, SE

1. Fusionsprotein, gekennzeichnet durch einen C- oder N-terminalen Anteil, der im wesentlichen den ersten 100 Aminosäuren von Interleukin-2 entspricht, aber keine Interleukin-2-Aktivität aufweist.

2. Fusionsprotein nach Anspruch 1 mit der allgemeinen Formel

$$\underset{(Ia)}{Met - X - Y - Z} \quad \text{oder} \quad \underset{(Ib)}{Met - Z - Y - X}$$

in der X im wesentlichen die Aminosäurefolge der etwa 100 ersten Aminosäuren des menschlichen Interleukin-2 bedeutet, Y eine direkte Bindung oder ein Brückenglied aus genetisch codierbaren Aminosäuren bedeutet, das die Abspaltung der Aminosäuresequenz Z ermöglicht, vorzugsweise, benachbart zu Z, Met, Cys, Trp, Arg oder Lys enthält oder aus diesen Aminosäuren besteht, insbesondere benachbart zu Z die Aminosäuresequenz

Asp - Pro,

enthält oder aus dieser Sequenz besteht und Z eine Sequenz aus genetisch codierbaren Aminosäuren ist, vorzugsweise von einem Proinsulin oder einem Hirudin.

3. Verfahren zur Herstellung eines Fusionsproteins nach Anspruch 1 oder 2, dadurch gekennzeichnet, daß man eine für dieses Protein codierende Genstruktur in einer Wirtszelle exprimiert und das Fusionsprotein, vorzugsweise durch Zentrifugation von den löslichen Proteinen, abtrennt.

4. Verfahren nach Anspruch 3, dadurch gekennzeichnet, daß die Wirtszelle ein Bakterium, vorzugsweise E. coli, ist.

5. Verwendung des Fusionsproteins nach Anspruch 2 bzw. der nach Anspruch 3 oder 4 erhaltenen Fusionsproteine zur Herstellung des Proteins, das im wesentlichen der Aminosäuresequenz Z entspricht, durch chemische oder enzymatische Spaltung.

6. Genstruktur, codierend für ein Fusionsprotein gemäß Anspruch 1 oder 2.

7. Vektor, enthaltend eine Genstruktur nach Anspruch 6.

8. Plasmide pEW 1000 (Fig. 1), pK360 (Fig. 2), pK410 (Fig. 2a), pPH30 (Fig. 3c), pK370 (Fig. 5a) und pKH101 (Fig. 6a).

9. Wirtszelle, enthaltend einen Vektor nach Anspruch 7.

**Patentansprüche für folgende Vertragsstaaten : ES, AT**

1. Verfahren zur Herstellung eines Fusionsproteins, dadurch gekennzeichnet, daß man in einer Wirtszelle ein Gen exprimiert, das für einen C- oder N-terminalen Anteil codiert, der im wesentlichen den ersten 100 Aminosäuren von Interleukin-2 (IL-2) entspricht, aber keine Interleukin-2-Aktivität aufweist.

2. Verfahren nach Anspruch 1, dadurch gekennzeichnet, daß das Gen für ein Fusionsprotein der allgemeinen Formel

$$\underset{(Ia)}{Met - X - Y - Z} \quad \text{oder} \quad \underset{(Ib)}{Met - Z - Y - X}$$

kodiert, in der X im wesentlichen die Aminosäurefolge der etwa 100 ersten Aminosäuren des menschlichen Interleukin-2 bedeutet, Y eine direkte Bindung oder ein Brückenglied aus genetisch codierbaren Aminosäuren bedeutet, das die Abspaltung der Aminosäuresequenz Z ermöglicht, und Z eine Sequenz aus genetisch codierbaren Aminosäuren ist.

3. Verfahren nach Anspruch 2, dadurch gekennzeichnet, daß Y, benachbart zu Z, Met, Cys, Trp, Arg oder Lys enthält oder aus diesen Aminosäuren besteht.

4. Verfahren nach Anspruch 2, dadurch gekennzeichnet, daß Y, benachbart zu Z, die Aminosäuresequenz

Asp - Pro,

enthält oder aus dieser Sequenz besteht.

5. Verfahren nach einem oder mehreren der vorhergehenden Ansprüche, dadurch gekennzeichnet, daß Z die Aminosäuresequenz von einem Proinsulin oder einem Hirudin bedeutet.

6. Verfahren nach einem oder mehreren der vorhergehenden Ansprüche, dadurch gekennzeichnet, daß das Fusionsprotein durch Zentrifugation von den löslichen Proteinen abgetrennt wird.

7. Verfahren nach einem oder mehreren der vorhergehenden Ansprüche, dadurch gekennzeichnet, daß die Wirtszelle ein Bakterium ist.

8. Verfahren nach Anspruch 7, dadurch gekennzeichnet, daß die Wirtszelle E. coli ist.

9. Verwendung der nach Anspruch 1 bis 8 erhaltenen Fusionsproteine zur Herstellung des Proteins, das im wesentlichen der Aminosäuresequenz Z entspricht, durch chemische oder enzymatische Spaltung.

**Claims**

**Claims for the following Contracting States : BE, CH, DE, FR, GB, GR, IT, LI, LU, NL, SE**

1. A fusion protein which has a C- or N-terminal section which essentially corresponds to the first 100 amino acids of interleukin-2 but does not have interleukin-2 activity.

2. A fusion protein as claimed in claim 1 with the formula

$$\text{Met} - X - Y - Z \quad \text{or} \quad \text{Met} - Z - Y - X$$
$$\text{(Ia)} \qquad\qquad\qquad \text{(Ib)}$$

in which

X essentially denotes the amino acid sequence of approximately the first 100 amino acids of human interleukin-2

Y denotes a direct bond or a bridging element which is composed of genetically codable amino acids and which allows the amino acid sequence Z to be cleaved off preferably contains, adjacent to Z, Met, Cys, Trp, Arg or Lys, or is composed of these amino acids, in particular contains, adjacent to Z, the amino acid sequence

Asp - Pro,

or is composed of this sequence, and

Z is a sequence of genetically codable amino acids, preferably of a proinsulin or of a hirudin.

3. A process for the preparation of a fusion protein as claimed in claim 1 or 2, which comprises expression of a gene structure coding for this protein in a host cell, and removal of the fusion protein, preferably by centrifugation from the soluble proteins.

4. The process as claimed in claim 3, wherein the host cell is a bacterium, preferably E. coli.

5. The use of the fusion protein as claimed in claim 2, or of the fusion proteins obtained as claimed in claim 3 or 4, for the preparation of the protein which essentially corresponds to the amino acid sequence Z by chemical or enzymatic cleavage.

6. A gene structure coding for a fusion protein as claimed in claim 1 or 2.

7. A vector containing a gene structure as claimed in claim 6.

8. Plasmids pEW 1000 (Fig. 1), pK360 (Fig. 2), pK410 (Fig. 2a), pPH30 (Fig. 3c), Pk370 (Fig. 5a) and pKH101 (Fig. 6a).

9. A host cell containing a vector as claimed in claim 7.

**Claims for the following Contracting States : ES, AT**

1. A process for the preparation of a fusion protein, which comprises expression in a host cell of a gene which codes for a C- or N-terminal section which essentially corresponds to the first 100 amino acids of interleukin-2 (IL-2), but does not have interleukin-2 activity.

2. The process as claimed in claim 1, wherein the gene codes for a fusion protein of the formula

$$\text{Met} - X - Y - Z \quad \text{or} \quad \text{Met} - Z - Y - X$$
$$\text{(Ia)} \qquad\qquad \text{(Ib)}$$

in which

- X essentially denotes the amino acid sequence of approximately the first 100 amino acids of human interleukin-2
- Y denotes a direct bond or a bridging element which is composed of genetically codable amino acids and which allows the amino acid sequence Z to be cleaved off and
- Z is a sequence of genetically codable amino acids.

**3.** The process as claimed in claim 2, wherein Y, adjacent to Z, contains Met, Cys, Trp, Arg or Lys, or is composed of these amino acids.

**4.** The process as claimed in claim 2, wherein Y, adjacent to Z, contains the amino acid sequence

Asp - Pro,

or is composed of this sequence.

**5.** The process as claimed in one or more of the preceding claims, wherein Z denotes the amino acid sequence of a proinsulin or of a hirudin.

**6.** The process as claimed in one or more of the preceding claims, wherein the fusion protein is removed from the soluble proteins by centrifugation.

**7.** The process as claimed in one or more of the preceding claims, wherein the host cell is a bacterium.

**8.** The process as claimed in claim 7, wherein the host cell is E. coli.

**9.** The use of the fusion proteins obtained as claimed in claims 1 to 8 for the preparation of the protein which essentially corresponds to the amino acid sequence Z, by chemical or enzymatic cleavage.

**Revendications**
**Revendications pour les Etats contractants suivants : BE, CH, DE, FR, GB, GR, IT, LI, LU, NL, SE**

**1.** Protéine de fusion, caractérisée par un segment C-ou N-terminal qui correspond essentiellement aux 100 premiers aminoacides de l'interleukine-2, mais ne présente pas d'activité d'interleukine-2.

**2.** Protéine de fusion selon la revendication 1, de formule générale

$$\text{Met} - X - Y - Z \quad \text{ou} \quad \text{Met} - Z - Y - X$$
$$\text{(Ia)} \qquad\qquad \text{(Ib)}$$

dans lesquelles formules X représente essentiellement la séquence d'aminoacides des environ 100 premiers aminoacides de l'interleukine-2 humaine, Y représente une liaison directe ou un chaînon pontant composé d'aminoacides génétiquement codables, qui permet la séparation de la séquence d'aminoacides Z, de préférence contient, adjacents à Z, Met, Cys, Trp, Arg ou Lys ou consiste en ces aminoacides, en particulier contient, adjacente à Z, la séquence d'aminoacides
Asp-Pro,
ou consiste en cette séquence, et Z est une séquence d'aminoacides génétiquement codables, de préférence d'une proinsuline ou d'une hirudine.

**3.** Procédé pour la production d'une protéine de fusion selon la revendication 1 ou 2, caractérisé en ce que l'on exprime dans une cellule hôte une structure génique codant pour cette protéine et on sépare la protéine de fusion, de préférence par centrifugation, d'avec les protéines solubles.

4. Procédé selon la revendication 3, caractérisé en ce que la cellule est une bactérie, de préférence E. coli.

5. Utilisation de la protéine de fusion selon la revendication 2 ou des protéines de fusion obtenues selon la revendication 3 ou 4, pour la production de la protéine, qui correspond essentiellement à la séquence d'aminoacides Z, par coupure chimique ou enzymatique.

6. Structure génique, codant pour une protéine de fusion selon la revendication 1 ou 2.

7. Vecteur contenant une structure génique selon la revendication 6.

8. Plasmides pEW 1000 (figure 1), pK 360 (figure 2), pK 410 (figure 2a), pPH 30 (figure 3c), pK 370 (figure 5a) et pKH 101 (figure 6a).

9. Cellule hôte, contenant un vecteur selon la revendication 7.

**Revendications pour les Etats contractants suivants : AT, ES**

1. Procédé pour la production d'une protéine de fusion, caractérisé en ce que l'on exprime dans une cellule hôte un gène qui code pour un segment C- ou N-terminal qui correspond essentiellement aux 100 premiers aminoacides de l'interleukine-2 (IL-2), mais ne présente pas d'activité d'interleukine-2.

2. Procédé selon la revendication 1, caractérisé en ce que le gène code pour une protéine de fusion de formule générale

$$\underset{(Ia)}{Met - X - Y - Z} \quad ou \quad \underset{(Ib)}{Met - Z - Y - X}$$

dans lesquelles formules X représente essentiellement la séquence d'aminoacides des environ 100 premiers aminoacides de l'interleukine-2 humaine, Y représente une liaison directe ou un chaînon pontant composé d'aminoacides génétiquement codables, qui permet la séparation de la séquence d'aminoacides Z, et Z est une séquence d'aminoacides génétiquement codables.

3. Procédé selon la revendication 2, caractérisé en ce que Y contient, adjacents à Z, Met, Cys, Trp, Arg ou Lys ou consiste en ces aminoacides.

4. Procédé selon la revendication 2, caractérisé en ce que Y contient, adjacente à Z, la séquence d'aminoacides
Asp-Pro,
ou consiste en cette séquence.

5. Procédé selon une ou plusieurs des revendications précédentes, caractérisé en ce que Z représente la séquence d'aminoacides d'une proinsuline ou d'une hirudine.

6. Procédé selon une ou plusieurs des revendications précédentes, caractérisé en ce que la protéine de fusion est séparée par centrifugation d'avec les protéines solubles.

7. Procédé selon une ou plusieurs des revendications précédentes, caractérisé en ce que la cellule hôte est une bactérie.

8. Procédé selon la revendication 7, caractérisé en ce que la cellule hôte est E. coli..

9. Utilisation de la protéine de fusion obtenue selon l'une des revendications 1 à 8, pour la production de la protéine qui correspond essentiellement à la séquence d'aminoacides Z, par coupure chimique ou

enzymatique.

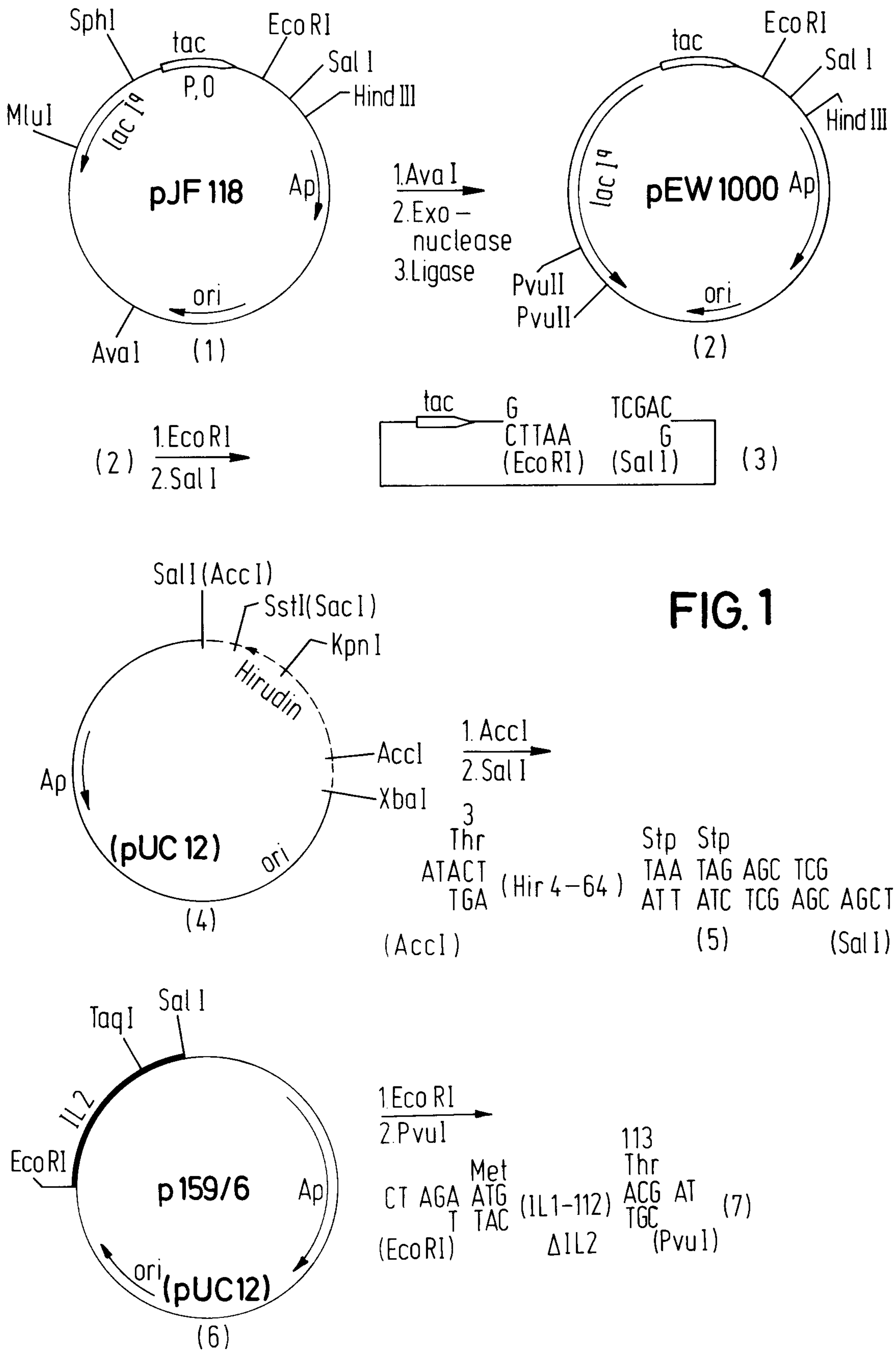
SphI
tac
P,0
EcoRI
Sal I
HindIII
MluI
lac I^q
pJF 118
Ap
ori
AvaI
(1)
1.AvaI
2.Exo-nuclease
3.Ligase
tac
EcoRI
Sal I
HindIII
lac I^q
pEW 1000
Ap
PvuII
PvuII
ori
(2)
(2)
1.EcoRI
2.Sal I
tac
G
CTTAA
(EcoRI)
TCGAC
G
(Sal I)
(3)
Sal I (Acc I)
SstI (Sac I)
KpnI
Hirudin
AccI
XbaI
Ap
(pUC 12)
ori
(4)
1. AccI
2. Sal I
3
Thr
ATACT
TGA
(Hir 4-64)
Stp Stp
TAA TAG AGC TCG
ATT ATC TCG AGC AGCT
(AccI)
(5)
(Sal I)
TaqI
Sal I
IL2
EcoRI
p 159/6
Ap
ori
(pUC12)
(6)
1.EcoRI
2.PvuI
113
Thr
Met
CT AGA ATG (IL1-112) ACG AT
T TAC TGC
(EcoRI)
ΔIL2
(PvuI)
(7)

FIG. 1

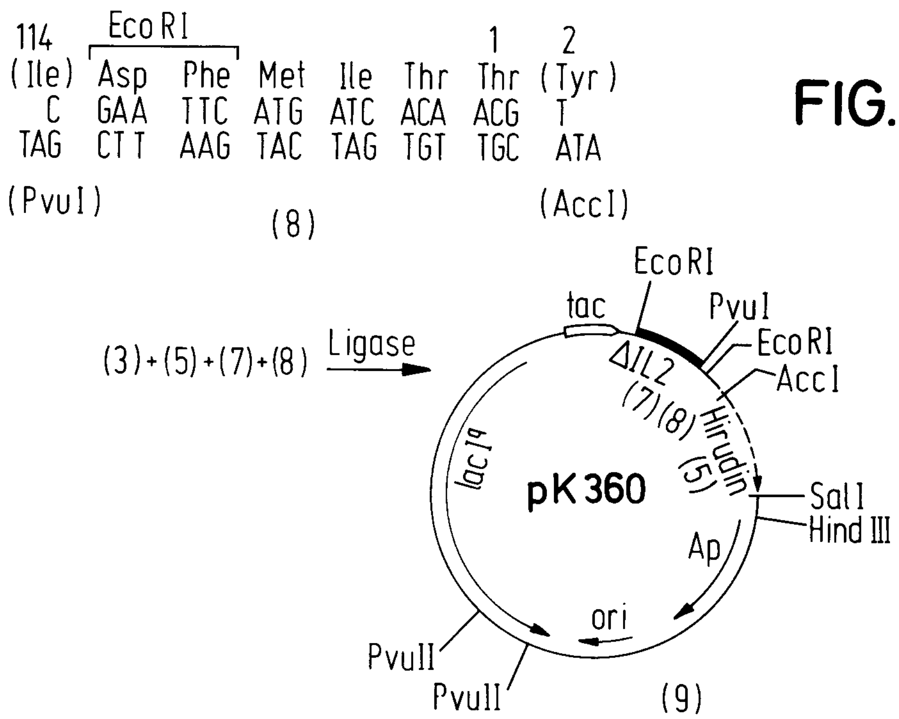
114
EcoRI
1
2
(Ile) Asp Phe Met Ile Thr Thr (Tyr)
C GAA TTC ATG ATC ACA ACG T
TAG CTT AAG TAC TAG TGT TGC ATA
(PvuI)
(8)
(AccI)
(3)+(5)+(7)+(8)
Ligase
tac
EcoRI
PvuI
EcoRI
AccI
ΔIL2
(7)(8)
Hirudin
(5)
lac I^q
pK360
SalI
Hind III
Ap
ori
PvuII
PvuII
(9)

FIG.1a

FIG.2

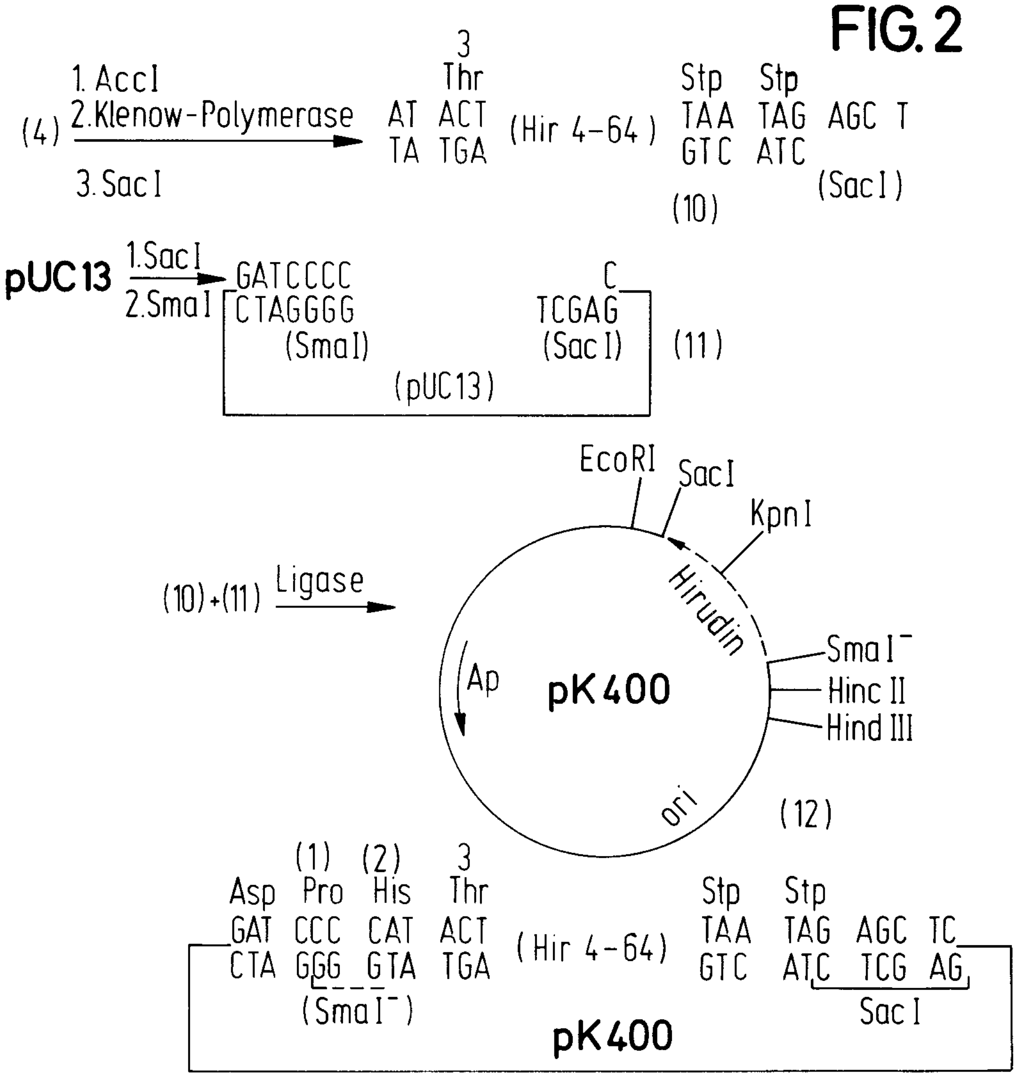
(4)
1. AccI
2. Klenow-Polymerase
3. SacI
3
Thr
AT ACT
TA TGA
(Hir 4–64)
Stp Stp
TAA TAG AGC T
GTC ATC
(SacI)
(10)
pUC13
1.SacI
2.SmaI
GATCCCC
CTAGGGG
(SmaI)
C
TCGAG
(SacI)
(pUC13)
(11)
(10)+(11)
Ligase
EcoRI
SacI
KpnI
Hirudin
SmaI⁻
Hinc II
Hind III
Ap
pK400
ori
(12)
(1) (2) 3
Asp Pro His Thr
GAT CCC CAT ACT
CTA GGG GTA TGA
(Hir 4–64)
Stp Stp
TAA TAG AGC TC
GTC ATC TCG AG
(SmaI⁻)
SacI
pK400

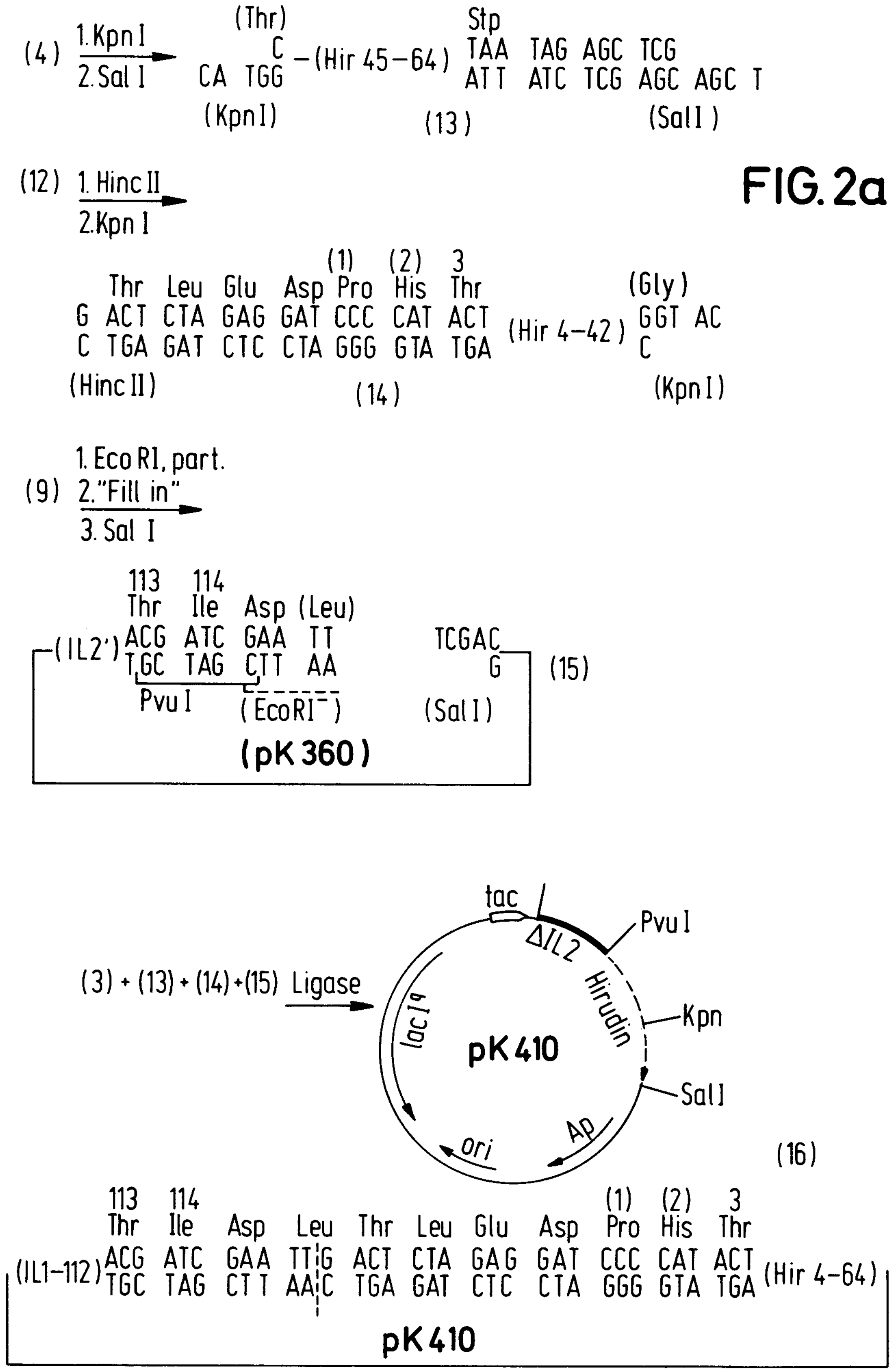
(4)
1. Kpn I
2. Sal I
(Thr)
C
CA TGG
–(Hir 45–64)
Stp
TAA TAG AGC TCG
ATT ATC TCG AGC AGC T
(KpnI)
(13)
(SalI)
(12)
1. Hinc II
2. Kpn I
(1) (2) 3
Thr Leu Glu Asp Pro His Thr
G ACT CTA GAG GAT CCC CAT ACT
C TGA GAT CTC CTA GGG GTA TGA
(Hir 4–42)
(Gly)
GGT AC
C
(Hinc II)
(14)
(KpnI)
1. Eco RI, part.
(9)
2. "Fill in"
3. Sal I
113 114
Thr Ile Asp (Leu)
–(IL2')
ACG ATC GAA TT
TGC TAG CTT AA
TCGAC
G
(15)
Pvu I
(EcoRI⁻)
(SalI)
(pK360)
tac
ΔIL2
Pvu I
Hirudin
Kpn
Sal I
lacI q
pK410
ori
Ap
(3) + (13) + (14) + (15) Ligase
(16)
113 114 (1) (2) 3
Thr Ile Asp Leu Thr Leu Glu Asp Pro His Thr
(IL1–112)
ACG ATC GAA TTG ACT CTA GAG GAT CCC CAT ACT
TGC TAG CTT AAC TGA GAT CTC CTA GGG GTA TGA
(Hir 4–64)
pK410

FIG. 2a

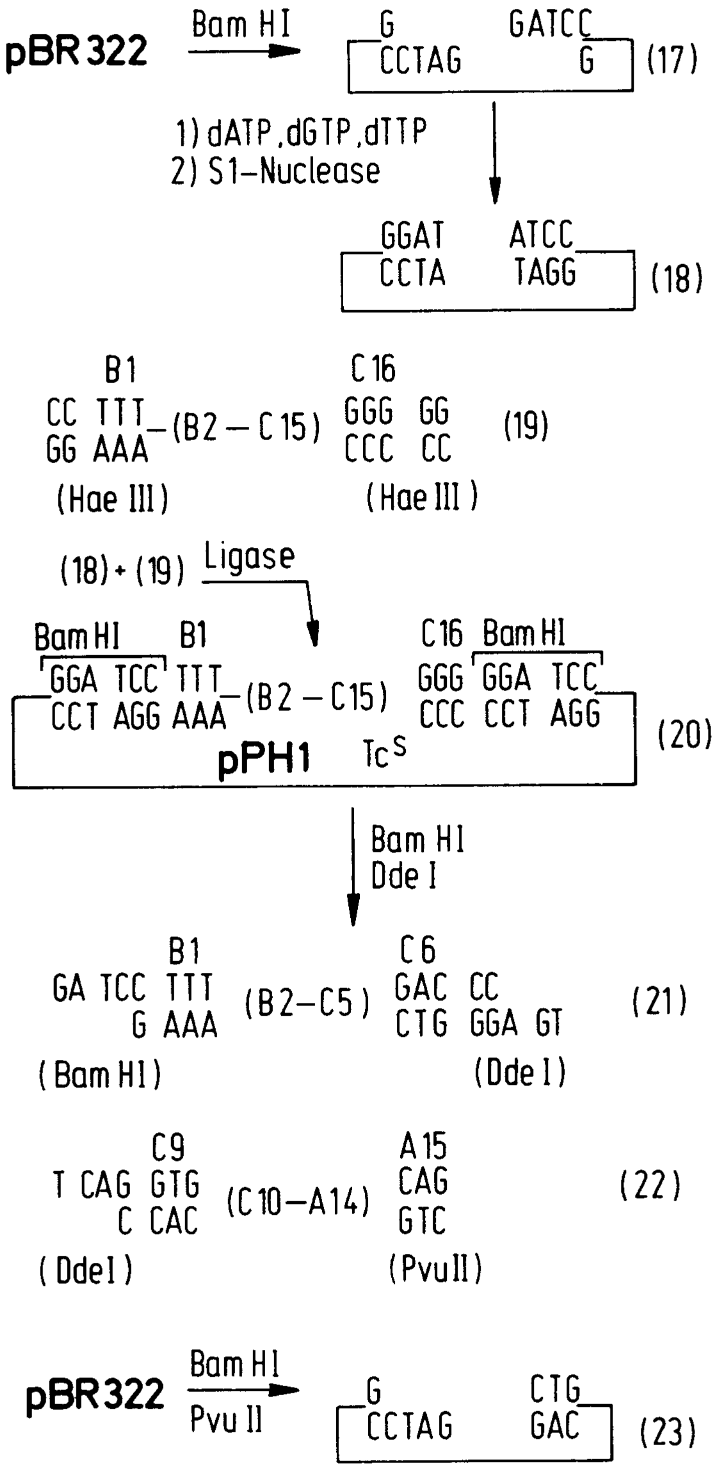
pBR322
Bam HI
G GATCC
CCTAG G
(17)
1) dATP,dGTP,dTTP
2) S1-Nuclease
GGAT ATCC
CCTA TAGG
(18)
B1
CC TTT
GG AAA
-(B2 – C15)
C16
GGG GG
CCC CC
(19)
(Hae III)
(Hae III)
(18)+(19)
Ligase
Bam HI
B1
GGA TCC TTT
CCT AGG AAA
-(B2 – C15)
C16
Bam HI
GGG GGA TCC
CCC CCT AGG
pPH1
Tc^S
(20)
Bam HI
Dde I
B1
GA TCC TTT
G AAA
(B2–C5)
C6
GAC CC
CTG GGA GT
(21)
(Bam HI)
(Dde I)
C9
T CAG GTG
C CAC
(C10–A14)
A15
CAG
GTC
(22)
(DdeI)
(PvuII)
pBR322
Bam HI
Pvu II
G CTG
CCTAG GAC
(23)

FIG. 3

**FIG. 3a**

(21)+(22)+(23) Ligase →

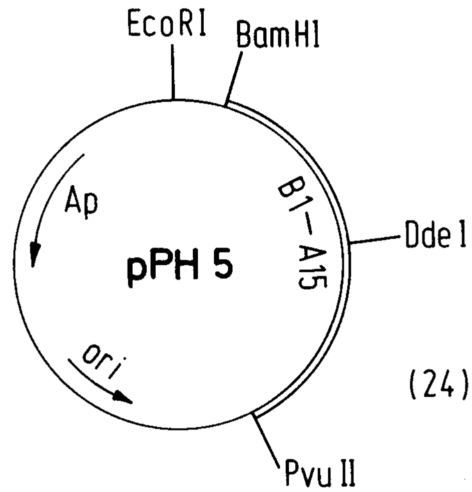


(24)

(24) BamHI / PvuII →

```
GATCC  (B1–A14)  A15          (25)
    G            CAG
                 GTC
(BamHI)          (PvuII)
```

```
A16                          A21  Stp  Stp
CTG  GAG  AAC  TAC  TGC  AAC  TAA  TAG                 (26)
GAC  CTC  TTG  ATG  ACG  TTG  ATT  ATC  AGC  T
(PvuII)                                (SalI)
```

**pUC 8** Bam HI / Sal I →

```
 (Gly)
 G            TCGAC
 CCT AG           G     (27)
 (BamHI)    (SalI)
```

(25)+(26)+(27) Ligase →

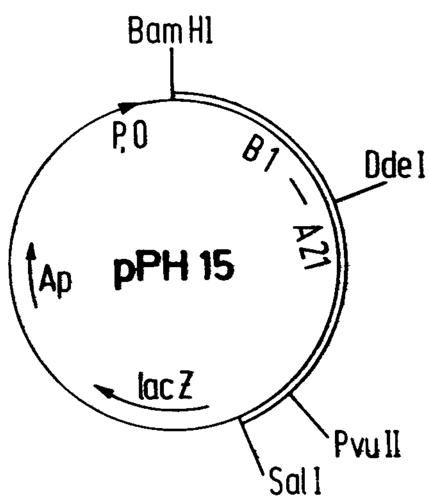


(28)

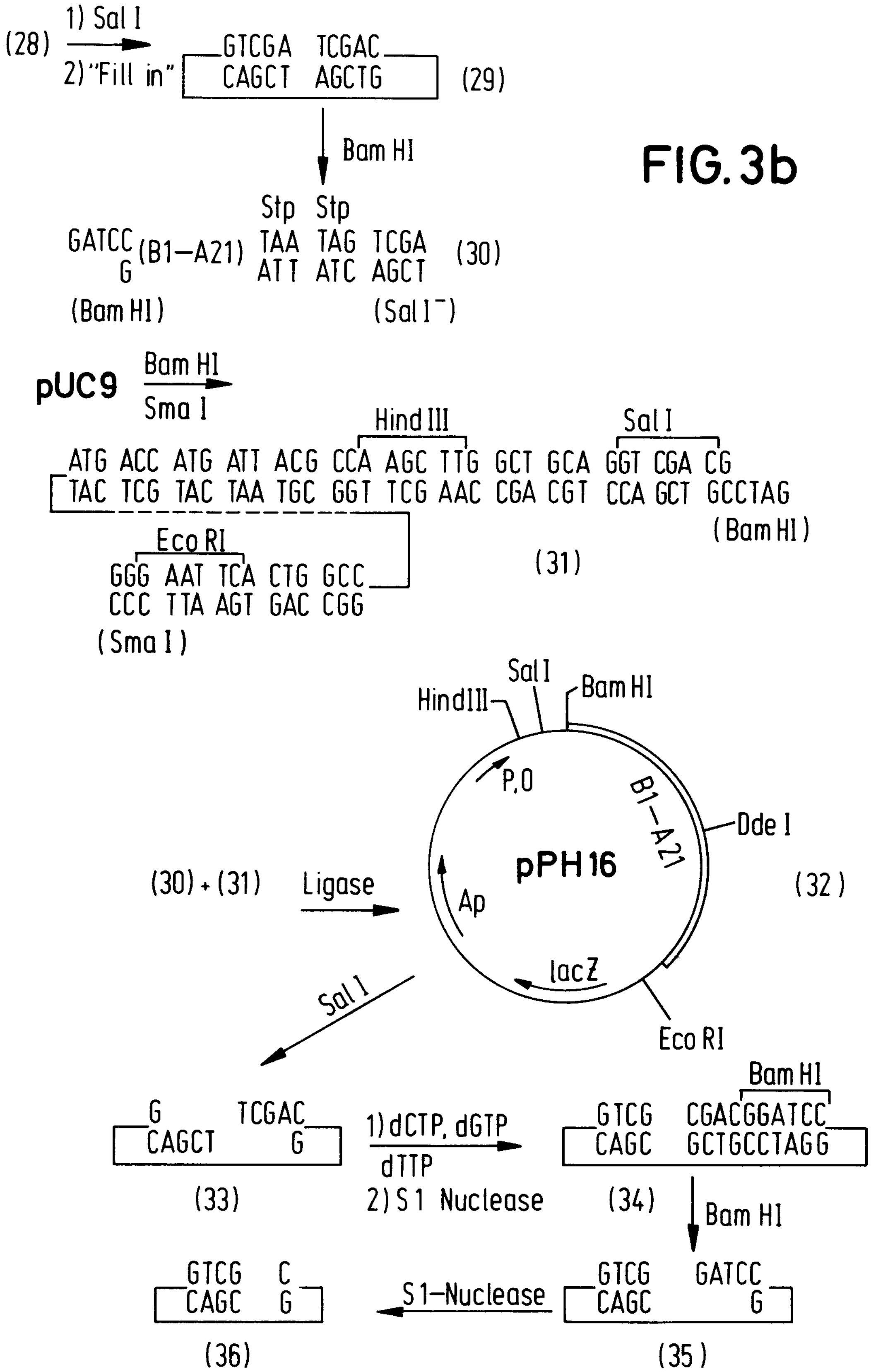
(28)
1) Sal I
2) "Fill in"
GTCGA TCGAC
CAGCT AGCTG
(29)
Bam HI
Stp Stp
GATCC (B1–A21) TAA TAG TCGA
G ATT ATC AGCT
(30)
(Bam HI)
(Sal I⁻)
pUC9
Bam HI
Sma I
Hind III
Sal I
ATG ACC ATG ATT ACG CCA AGC TTG GCT GCA GGT CGA CG
TAC TCG TAC TAA TGC GGT TCG AAC CGA CGT CCA GCT GCCTAG
(Bam HI)
Eco RI
GGG AAT TCA CTG GCC
CCC TTA AGT GAC CGG
(Sma I)
(31)
Sal I
Bam HI
Hind III
P,O
B1–A21
Dde I
pPH16
Ap
lacZ
Eco RI
(30) + (31)
Ligase
(32)
Sal I
G TCGAC
CAGCT G
(33)
1) dCTP, dGTP
dTTP
2) S1 Nuclease
Bam HI
GTCG CGACGGATCC
CAGC GCTGCCTAGG
(34)
Bam HI
GTCG GATCC
CAGC G
(35)
S1–Nuclease
GTCG C
CAGC G
(36)

FIG. 3b

FIG. 3c

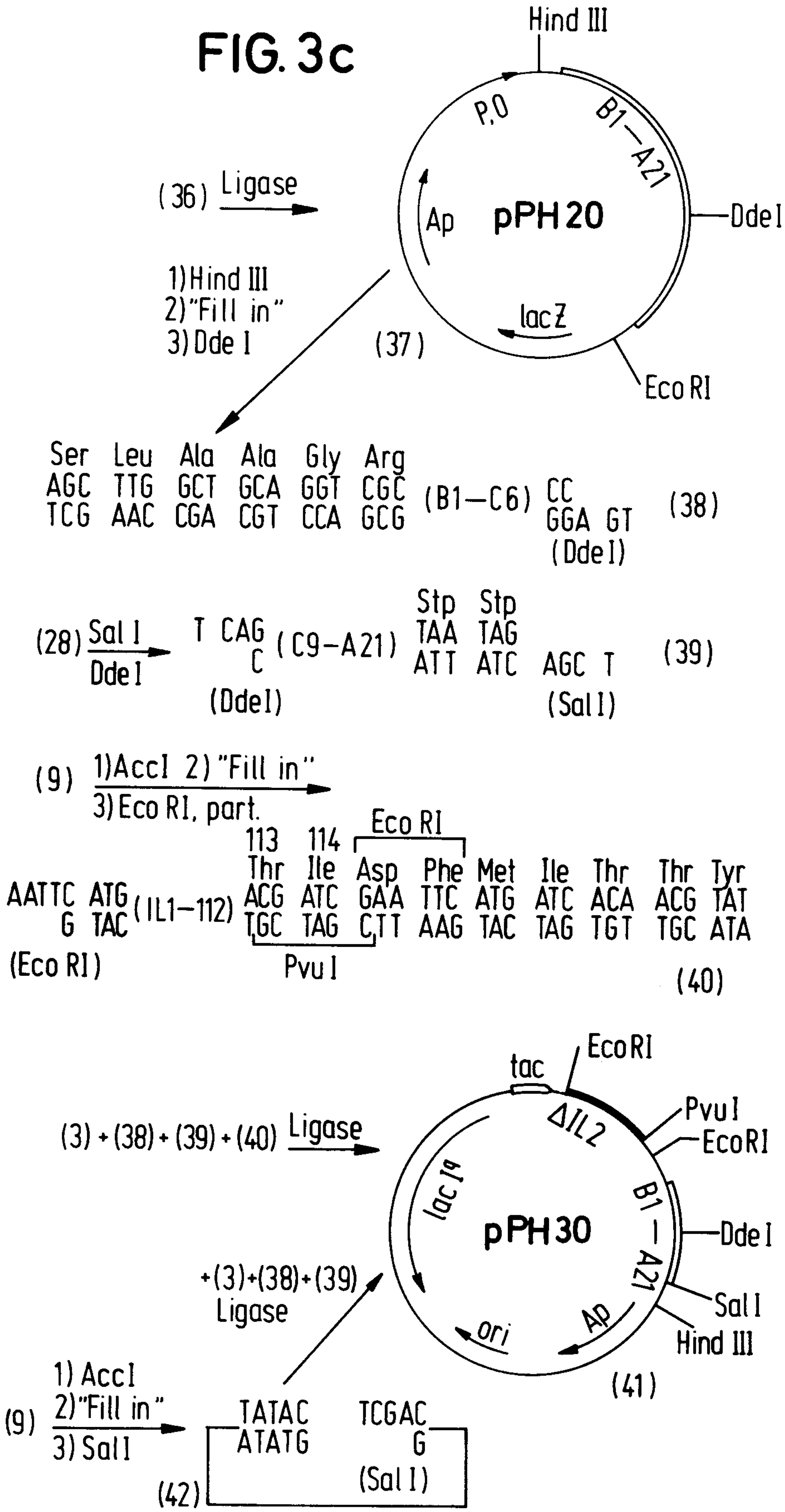
Hind III
P,O
B1—A21
(36) Ligase
Ap
pPH20
DdeI
1) Hind III
2) "Fill in"
3) Dde I
lacZ
(37)
Eco RI
Ser Leu Ala Ala Gly Arg
AGC TTG GCT GCA GGT CGC (B1—C6) CC
TCG AAC CGA CGT CCA GCG GGA GT (38)
(DdeI)
(28) Sal I / DdeI
T CAG (C9—A21) Stp Stp TAA TAG
C ATT ATC AGC T (39)
(DdeI) (SalI)
(9) 1) AccI 2) "Fill in" 3) Eco RI, part.
Eco RI
113 114
Thr Ile Asp Phe Met Ile Thr Thr Tyr
AATTC ATG (IL1—112) ACG ATC GAA TTC ATG ATC ACA ACG TAT
G TAC TGC TAG CTT AAG TAC TAG TGT TGC ATA
(Eco RI) PvuI (40)
tac
EcoRI
ΔIL2
PvuI
EcoRI
(3)+(38)+(39)+(40) Ligase
lac I^q
pPH30
B1—A21
DdeI
+(3)+(38)+(39) Ligase
SalI
ori
Ap
Hind III
(41)
(9) 1) AccI 2) "Fill in" 3) SalI
TATAC TCGAC
ATATG G
(SalI)
(42)

FIG. 4

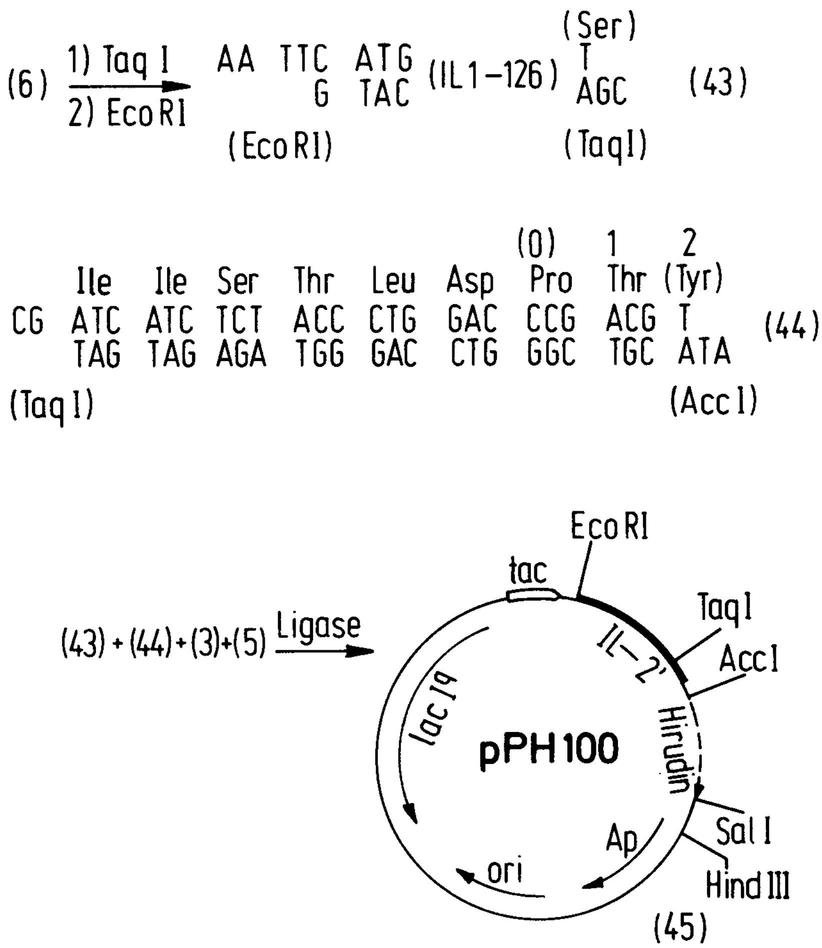
(6)
1) Taq I
2) EcoRI
AA TTC ATG
G TAC
(EcoRI)
(IL1–126)
(Ser)
T
AGC
(TaqI)
(43)
(0) 1 2
Ile Ile Ser Thr Leu Asp Pro Thr (Tyr)
CG ATC ATC TCT ACC CTG GAC CCG ACG T
TAG TAG AGA TGG GAC CTG GGC TGC ATA
(Taq I)
(Acc I)
(44)
(43) + (44) + (3) + (5)
Ligase
EcoRI
tac
TaqI
AccI
IL–2'
Hirudin
lacIq
pPH100
Ap
ori
Sal I
Hind III
(45)

FIG. 5

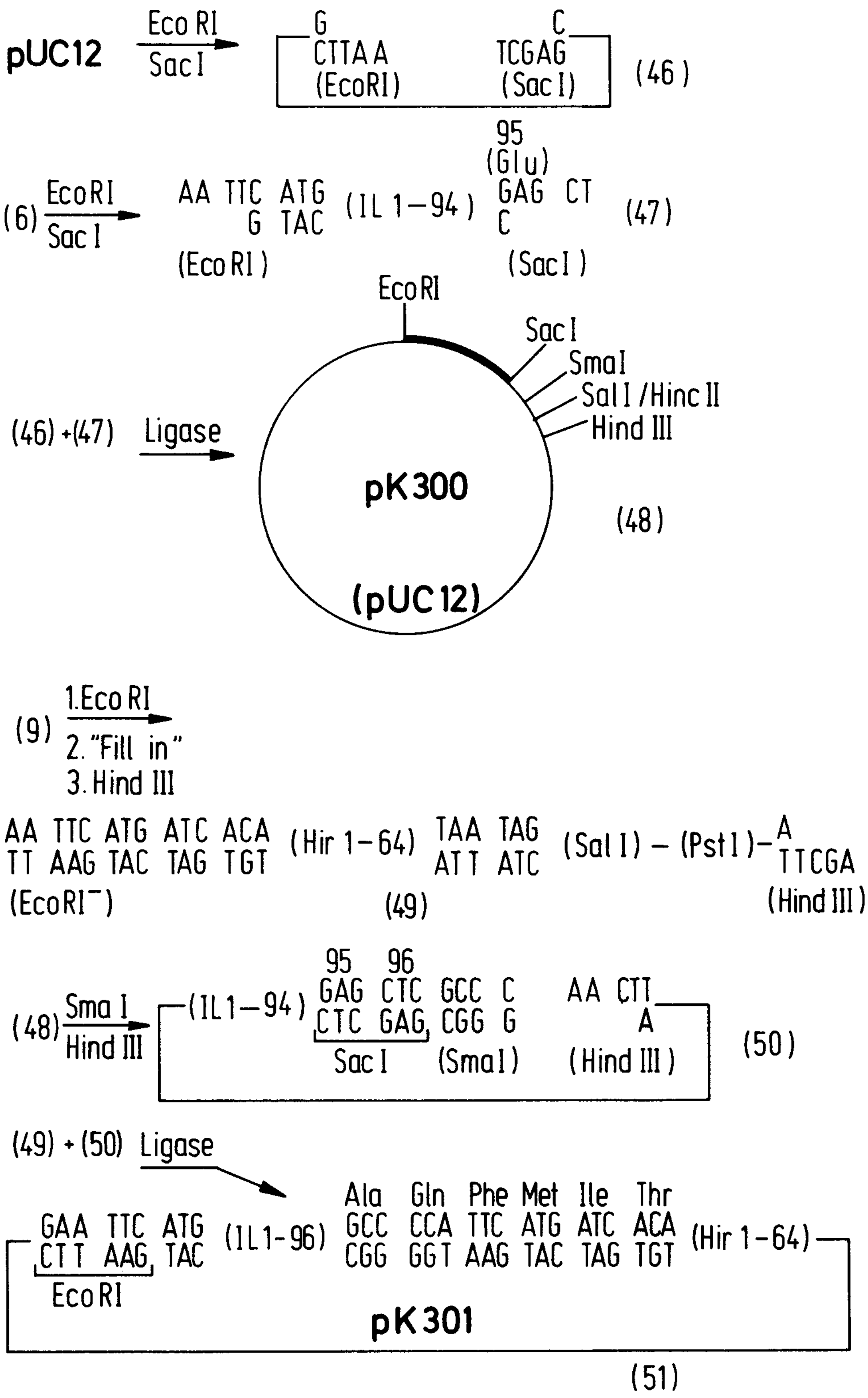

pUC12
Eco RI
Sac I
G
CTTAA
(EcoRI)
C
TCGAG
(Sac I)
(46)
95
(Glu)
(6)
EcoRI
Sac I
AA TTC ATG
G TAC
(IL 1-94)
GAG CT
C
(47)
(EcoRI)
(SacI)
EcoRI
SacI
SmaI
SalI/HincII
Hind III
(46)+(47) Ligase
pK300
(pUC12)
(48)
(9)
1. Eco RI
2. "Fill in"
3. Hind III
AA TTC ATG ATC ACA
TT AAG TAC TAG TGT
(Hir 1-64)
TAA TAG
ATT ATC
(Sal I) - (Pst I)-
A
TTCGA
(EcoRI⁻)
(49)
(Hind III)
95 96
(48)
Sma I
Hind III
-(IL1-94)
GAG CTC GCC C
CTC GAG CGG G
AA CTT
A
Sac I
(SmaI)
(Hind III)
(50)
(49) + (50) Ligase
Ala Gln Phe Met Ile Thr
GAA TTC ATG
CTT AAG TAC
(IL1-96)
GCC CCA TTC ATG ATC ACA
CGG GGT AAG TAC TAG TGT
(Hir 1-64)
EcoRI
pK301
(51)

FIG.5a

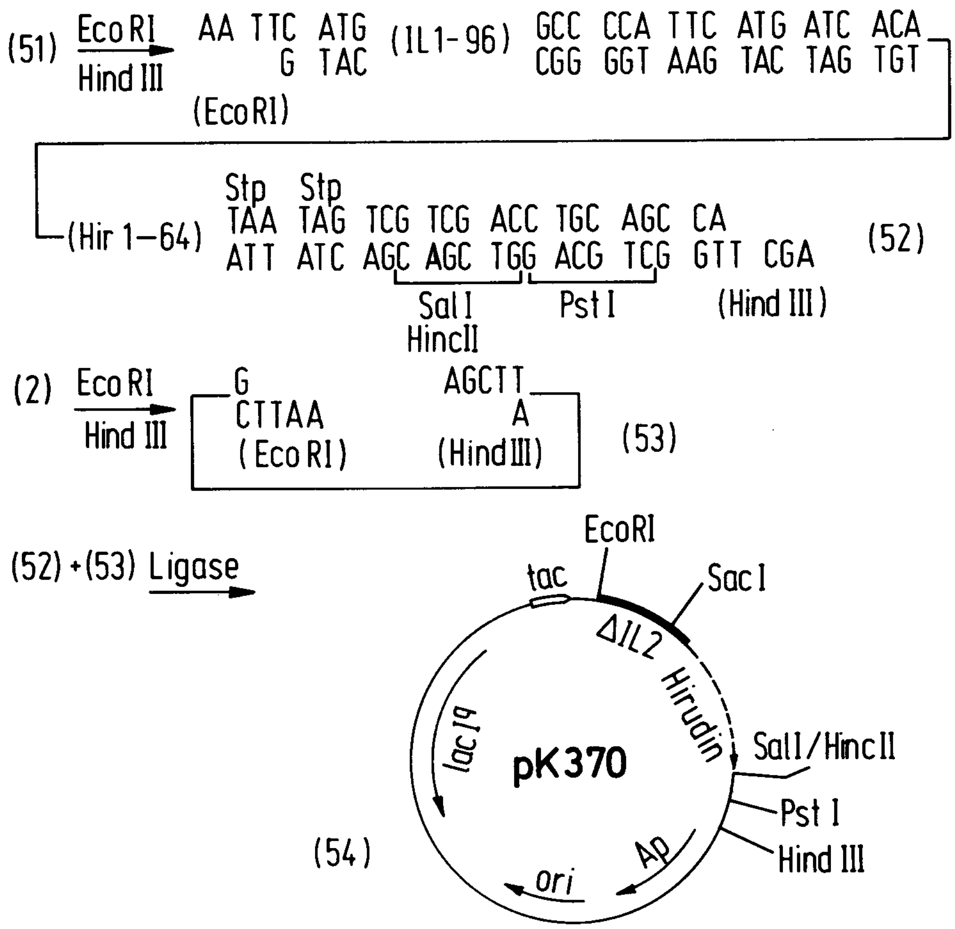
(51) EcoRI Hind III AA TTC ATG G TAC (EcoRI) (IL1-96) GCC CCA TTC ATG ATC ACA CGG GGT AAG TAC TAG TGT
(Hir 1-64) Stp Stp TAA TAG TCG TCG ACC TGC AGC CA ATT ATC AGC AGC TGG ACG TCG GTT CGA (52)
SalI HincII PstI (Hind III)
(2) EcoRI Hind III G CTTAA (EcoRI) AGCTT A (HindIII) (53)
(52)+(53) Ligase
EcoRI
SacI
tac
ΔIL2
Hirudin
lacIq
pK370
SalI/HincII
PstI
Hind III
Ap
ori
(54)

FIG. 6

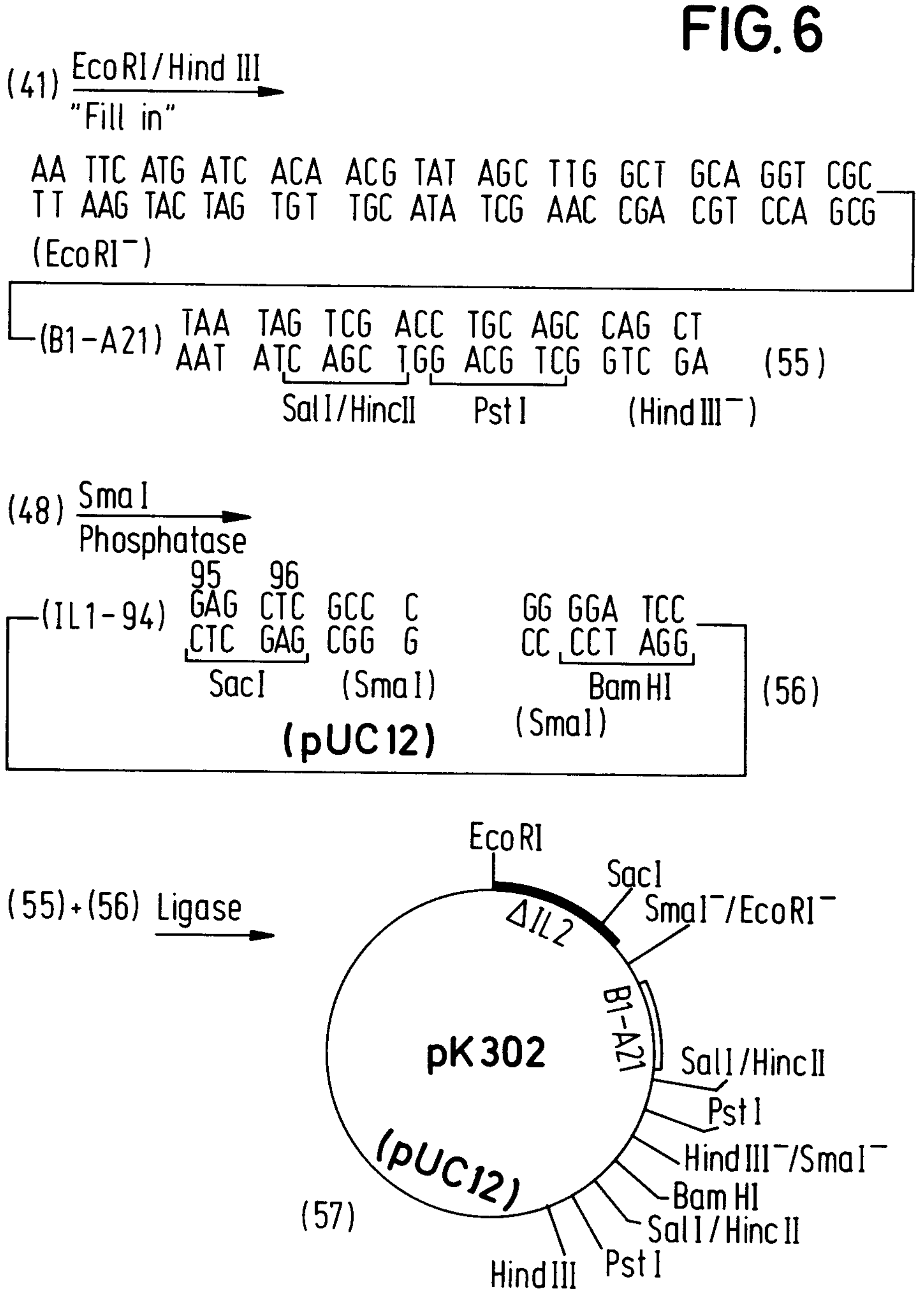
(41) EcoRI/Hind III
"Fill in"
AA TTC ATG ATC ACA ACG TAT AGC TTG GCT GCA GGT CGC
TT AAG TAC TAG TGT TGC ATA TCG AAC CGA CGT CCA GCG
(EcoRI⁻)
(B1-A21) TAA TAG TCG ACC TGC AGC CAG CT
AAT ATC AGC TGG ACG TCG GTC GA
SalI/HincII
PstI
(HindIII⁻)
(55)
(48) SmaI
Phosphatase
95 96
(IL1-94) GAG CTC GCC C
CTC GAG CGG G
SacI
(SmaI)
GG GGA TCC
CC CCT AGG
BamHI
(SmaI)
(pUC12)
(56)
(55)+(56) Ligase
EcoRI
SacI
SmaI⁻/EcoRI⁻
ΔIL2
B1-A21
pK302
SalI/HincII
PstI
HindIII⁻/SmaI⁻
BamHI
SalI/HincII
PstI
HindIII
(pUC12)
(57)

FIG. 6a

(57) $\xrightarrow[\text{Hind III}]{\text{Eco RI}}$ EcoRI (IL1–96)–Y–(B1–A21)–Hind III (58)

Y=

| | | | | | | | | | | | | | |
|---|---|---|---|---|---|---|---|---|---|---|---|---|---|
| Ala | Gln | Phe | Met | Ile | Thr | Thr | Tyr | Ser | Leu | Ala | Ala | Gly | Arg |
| GCC | CAA | TTC | ATG | ATC | ACA | ACG | TAT | AGC | TTG | GCT | GCA | GGT | CGC |
| CGG | GTT | AAG | TAC | TAG | TGT | TGC | ATA | TCG | AAC | CGA | GGT | CCA | GCG |

(58) + (53) $\xrightarrow{\text{Ligase}}$